# EUROPEAN PATENT APPLICATION

(11) **EP 3 124 955 A1**
(43) Date of publication of application: **01.02.2017**
(21) Application number: 15179433.6
(22) Date of filing: 31.07.2015
(51) Int. Cl.: G01N 21/3577, G01N 33/12

(54) **RAPID DIFFERENTIATION OF FRESH AND FROZEN/THAWED MEAT SAMPLES BY FT-IR ON LIQUID MEAT SAMPLES**

(71) Applicant: Universität Zürich, 8006 Zürich (CH); Veterinärmedizinische Universität Wien, 1210 Vienna (AT)
(72) Inventor: EHLING-SCHULZ, Monika, 1220 Wien (AT); GRUNERT, Tom, 1140 Wien (AT); STEPHAN, Roger, 6042 Dietwil (CH); JOHLER-ILIC, Sophia, 8057 Zürich (CH)
(74) Representative: Schulz Junghans Patentanwälte PartGmbB

(57) **Abstract**

The invention relates to a method for analysing a meat sample, wherein the method comprises providing a meat sample, and obtaining a liquid sample from the meat sample, measuring a sample optical value of the liquid sample in a spectral range of 4000 cm⁻¹ to 400 cm⁻¹ in a spectroscopy step, determining, in a comparison step, particularly in a computing step, a similarity value by comparing the sample optical value to at least one control optical value obtained from at least one control sample, wherein the control sample has been prepared from fresh meat, and/or from frozen and thawed meat, and assigning the meat sample to "fresh meat" or "frozen and thawed meat" based on the similarity value.

## Description

The present invention relates to a method for differentiating fresh meat and frozen/thawed meat by spectroscopy, particularly FTIR-spectroscopy or RAMAN spectroscopy.

Chicken is a highly perishable meat product. Freezing and frozen storage of chicken and other meat products is a common practice used in the striving global meat export industry, which is currently worth more than 13 billion USD. According to the Food and Agriculture Organization of the United Nations, freezing of poultry can extend the practical storage life to up to two years. However, freezing and thawing negatively affects the sensory profile and therefore the quality of meat through formation of ice crystals, oxidation of lipids and degradation of proteins, reduced tenderness, and reduced water holding capacity. The impaired quality of frozen/thawed chicken and the resulting impact on marketability is also reflected in lower pricing. In December 2014, the EU implemented a new regulation specifying that frozen/thawed products have to be labeled "defrosted", as safety, taste and the physical quality of food items - in particular meat - can be affected. However, fresh and frozen/thawed chicken meat are virtually indistinguishable. In addition, the currently available testing methods for differentiation of fresh and frozen/thawed chicken are laborious, time consuming, and cost intensive. Consequently, retailers often rely on information provided by sub-contractors, which bears the risk of mislabeling and its negative impact on the consumers' repurchase behavior. Therefore, a novel high-throughput tool suited for reliable differentiation of fresh and frozen/thawed chicken is urgently needed.

Various techniques for the differentiation of fresh and frozen/thawed meat products have been described. Methods include enzymatic tests, optical microscopy, measurement of the K-value, evaluation of drip loss, and pH measurements. However, to date, no rapid, reliable, and inexpensive technique for the identification of frozen/thawed chicken has been successfully implemented. Differentiation of fresh and frozen/thawed products is of high interest to retailers not only in the case of poultry, but also for all other kinds of meat.

Thus, the objective underlying the present invention is the provision of a simple, reliable method for determining the freshness of meat.

This objective is attained by the subject matter of the independent claim.

According to a first aspect of the invention, a method for analysing a meat sample is provided. The method comprises providing a meat sample, obtaining a liquid sample from the meat sample, measuring a sample optical value of the liquid sample in a spectral range of 4000 cm⁻¹ to 400 cm⁻¹ in a spectroscopy step, determining, in a comparison step, particularly in a computing step, a similarity value by comparing the sample optical value to at least one control optical value, wherein the at least one control optical value is obtained from a liquid control sample, which is prepared in the same way as the liquid sample, the control optical value being determined at the same wave number as the sample optical value, wherein the at least one control sample has been prepared from fresh meat, and/or from frozen/thawed (frozen and thawed) meat, and assigning the meat sample to "fresh meat" or "frozen and thawed meat" based on the similarity value. Therein, the sample optical value and the at least one control optical value are determined using the same parameters during the acquisition and processing of data.

Advantageously, the use of a liquid sample reduces the complexity of the resulting spectra.

In the context of the present specification, the term meat describes mammalian or avian tissue, particularly chicken tissue.

In certain embodiments, the meat sample is obtained from muscle tissue.

In particular, a liquid sample prepared from a meat sample may comprise tissue liquid and/or cells and/or an extract of cells and/or collagen.

The spectral range of wave numbers from 4000 cm⁻¹ to 400 cm⁻¹ comprises the amide I, amide II, amide III, and amide A/B infrared bands of polypeptides.

In the context of the present specification, the term control sample describes a liquid sample prepared from a meat sample, wherein the meat sample has been provided either from fresh meat or from frozen and thawed meat. By comparing a sample optical value of a liquid sample from a meat sample of interest to one or more control optical values, wherein each control optical value is obtained from a control sample, and the sample optical value and the control optical value have been determined at the same wave number and using the same parameters during acquisition and processing of data, it can be decided whether the meat sample of interest has been provided from fresh meat or frozen/thawed meat.

In certain embodiments, the similarity value is a metric value, particularly a real number or a natural number, and the similarity value is determined by comparing a sample optical value of a sample of interest to one or more control optical values of a first plurality of control samples from fresh meat or to one or more control optical values of a second plurality of control samples prepared from frozen/thawed meat, particularly wherein the similarity value is larger if the sample optical value is more similar to the control optical values of the first plurality of control samples or if the sample optical value is more similar to the control optical values of the second plurality of control samples.

In certain embodiments, the first plurality of control optical values and the second plurality of control optical values are stored as sets of data. In this manner, the control optical values may be used several times to be compared to sample optical values of different samples of interest if the sample optical value is determined within the same spectral range and using the same parameters as the control optical values.

In certain embodiments, the similarity value is determined by calculating a statistical parameter of the control optical values, particularly a univariate statistical parameter, more particularly a mean or a median, or a multivariate statistical parameter, and comparing a sample optical value to the statistical parameter.

In certain embodiments, the similarity value between control optical values and sample optical values is determined by principal component analysis (PCA) or discriminant function analysis (Grunert et al J Clin Microbiol. 51(7), 2013 and Grunert et al., PLoS One 9(12), 2014), hierarchical cluster analysis (HCA), or an Artificial Neural Network (ANN, Udelhoven, Novozhilov, & Schmitt, Chemometrics and Intelligent Laboratory Systems 66, 2003).

In certain embodiments, the similarity value comprises a metric variable, particularly a real number or a natural number, and/or a categorical variable, particularly a category having the values "fresh", "frozen and thawed", or "no result".

In certain embodiments, a sample spectrum is determined in the spectroscopy step, particularly measured at wave numbers from 4000 cm⁻¹ to 400 cm⁻¹, wherein particularly additionally an at least one corresponding control spectrum of the liquid control sample is determined or measured within the same spectral range and using the same parameters as the sample spectrum.

In certain embodiments, a sample infrared spectrum is determined, particularly measured, at wave numbers from 4000 cm⁻¹ to 400 cm⁻¹ in the spectroscopy step.

The term "spectrum" in the context of the present specification is used in its meaning known in the art of physics. It refers to a plurality of optical values determined from a sample, particularly from the above-mentioned liquid sample or control sample, wherein the optical values are determined within a range of wavelengths or wave numbers.

In certain embodiments, the spectrum is a continuous spectrum consisting of a plurality of optical values that are determined at uniformly distributed wavelengths or wave numbers within the above or below mentioned spectral range.

In certain embodiments, the optical value, particularly the sample optical value or the control optical value, is an absorption value of the liquid sample.

In certain embodiments, the sample optical value and the control optical value are determined by obtaining a transmittance value or an absorbance value, preferably the transmittance value of the liquid sample. In certain embodiments, the sample spectrum and the control spectrum are determined by obtaining transmittance or absorbance values, preferably transmittance values of the liquid sample.

In the context of the present specification, the term transmittance value describes the ratio between a light intensity, which is measured after the light has passed through a sample and an initial light intensity before the light has entered the sample. In particular, a transmittance value may be determined by dividing the light intensity after the light has passed through the sample by the initial light intensity.

In the context of the present specification, the term absorbance value describes the logarithm to the base 10 of the ratio between an initial light intensity and a light intensity after the light has passed through a sample.

Advantageously, the determination of an optical value or a spectrum by measurement of transmittance values instead of measuring light reflected from a sample, particularly by Attenuated Total Reflectance (ATR) measurement, enables a high-throughput measurement of samples.

For example, a 96-well microplate sample holder may be used to perform the method described herein, which allows a high-throughput screening of dried samples in the absorbance mode. Compared to methods of the prior art, in which wet samples were measured using the ATR method and single sample holders were used, which had to be cleaned between each measurement, the method disclosed herein allows faster measurement with a potential application in routine diagnostics.

In certain embodiments, the optical value or the spectrum is determined by Fourier-transformed infrared spectroscopy (FTIR) or Attenuated Total Reflection (ATR) spectroscopy.

According to a preferred embodiment of the invention, Fourier-transformed infrared spectroscopy (FTIR) is used to determine the sample optical value and the control optical value in the spectroscopy step. According to a further preferred embodiment of the invention, Fourier-transformed infrared spectroscopy (FTIR) is used to determine the sample spectrum and the control spectrum in the spectroscopy step.

In certain embodiments, determining an optical value comprises measuring a light intensity of a specific wave number or measuring a light intensity within a spectral range of wave numbers. In certain embodiments, determining a spectrum comprises measuring light intensities of specific wave numbers or measuring light intensities within a spectral range of wave numbers.

According to a preferred embodiment of the invention, determining or measuring an optical value or a spectrum comprises measuring an interferogram.

In the context of the present specification, the term interferogram is used in its meaning known in the art of physics. Therein, determining an interferogram comprises measuring a light intensity of two interfering light beams having a different path length, after the light beams have passed through a sample or have been reflected by a sample.

In certain embodiments, the optical value is a scattering value of the liquid sample, wherein the spectral range from 4000 cm⁻¹ to 400 cm⁻¹ refers to the Raman shift of the inelastically scattered light, which has been scattered by the sample. In certain embodiments, a Raman sample spectrum of the liquid sample is determined or measured in the spectral range from 4000 cm⁻¹ to 400 cm⁻¹, wherein the Raman sample spectrum comprises a plurality of scattering values, and wherein particularly a Raman control spectrum of the liquid control sample is determined or measured within the same spectral range and using the same parameters as the sample spectrum.

The scattering value may be measured by Raman spectroscopy using a device comprising a light source, particularly a laser, more particularly a continuous wave argon laser or a continuous wave krypton laser, or an infrared diode laser with a wavelength of 785 nm, which is focused on the sample. Most particularly, second harmonic generation (SHG) may be used to double the frequency of the laser, in order to advantageously generate a larger amount of scattered light and/or for excitation of the sample in resonance Raman spectroscopy (Chi et al., Biochemistry 37, 1998). The device may further comprise a detector for detecting scattered light in an angle from 90° to 180°, particularly 135° or 180°, and a refractive or diffractive element for performing a spectral decomposition of the scattered light. In particular, the spectrum of scattering values in a spectral range of wavelengths or wave numbers may be measured by the device. Therein, the scattering value comprises the intensity of the scattered light.

In the context of the present specification, the term Raman shift (in a unit of a wave number, particularly cm⁻¹) for a particular scattering value designates the arithmetic difference of the inverse of the wavelength of the respective Rayleigh peak resulting from the elastically scattered light of the light source and the inverse of the wavelength at which the particular scattering value was measured. Therefore, the Raman shift represents the Stokes shift of the inelastically scattered light, which may correspond to the optical values determined by FTIR spectroscopy or ATR infrared spectroscopy.

In certain embodiments, the optical value, particularly the sample optical value, is determined in the spectral range of 3800 cm⁻¹ to 400 cm⁻¹, corresponding to wavelengths from 2.63 micrometres to 25 micrometres, particularly 1800 cm⁻¹ to 1000 cm⁻¹, corresponding to wavelengths from 5.56 micrometres to 10 micrometres, and/or 3500 cm⁻¹ to 3060 cm⁻¹, corresponding to wavelengths from 2.85 micrometres to 3.27 micrometres.

In certain embodiments, the optical value, particularly the sample optical value, is determined in the spectral range of 1800 cm⁻¹ to 1000 cm⁻¹, and an additional optical value, particularly an additional sample optical value, is determined in the spectral range of 3500 cm⁻¹ to 3060 cm⁻¹.

In certain embodiments, the sample spectrum is determined in the spectral range of 3800 cm⁻¹ to 400 cm⁻¹, corresponding to wavelengths from 2.63 micrometres to 25 micrometres, particularly 1800 cm⁻¹ to 1000 cm⁻¹, corresponding to wavelengths from 5.56 micrometres to 10 micrometres, and/or 3500 cm⁻¹ to 3060 cm⁻¹, corresponding to wavelengths from 2.85 micrometres to 3.27 micrometres.

In certain embodiments, the sample spectrum is determined in the spectral range of 1800 cm⁻¹ to 1000 cm⁻¹, and an additional sample spectrum is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, corresponding to wavelengths from 5.92 micrometres to 6.17 micrometres, particularly 1660 cm⁻¹ to 1628 cm⁻¹, corresponding to wavelengths from 6.02 micrometres to 6.14 micrometres and/or 1570 cm⁻¹ to 1515 cm⁻¹, corresponding to wavelengths from 6.37 micrometres to 6.60 micrometres, and/or 1440 cm⁻¹ to 1340 cm⁻¹, corresponding to wavelengths from 6.94 to 7.46 micrometres, and/ or 1305 cm⁻¹ to 1200 cm⁻¹, corresponding to wavelengths from 7.66 micrometres to 8.33 micrometres.

Preferably, the optical value, particularly the sample optical value, is determined in the spectral range of 1690 cm⁻¹ to 1620 cm⁻¹, more preferably 1660 cm⁻¹ to 1628 cm⁻¹, and/or in the spectral range of 1570 cm⁻¹ to 1515 cm⁻¹, and/or 1305 cm⁻¹ to 1200 cm⁻¹, and/or 1440 cm⁻¹ to 1340 cm⁻¹, and/or 3500 cm⁻¹ to 3060 cm⁻¹.

In certain embodiments, the optical value, particularly the sample optical value, is determined in the spectroscopy step within at least one spectral range of wave numbers, wherein the spectral range is 1690 cm⁻¹ to 1620 cm⁻¹, comprising the amide I band of polypeptides and/or 1570 cm⁻¹ to 1515 cm⁻¹, comprising the amide II band of polypeptides, and/or 1440 cm⁻¹ to 1340 cm⁻¹, comprising the band attributed to C_{α}-H amide bending vibrations (Chi et al., *Biochemistry* **37,** 1998; Overman & Thomas, Biochemistry 37, 1998), and/or 1305 cm⁻¹ to 1200 cm⁻¹, comprising the amide III band of polypeptides and/or 3500 cm⁻¹ to 3060 cm⁻¹, comprising the amide A/B band of polypeptides.

These infrared bands are sensitive to the secondary structure of polypeptides and therefore display differential intensities in samples comprising native and denatured polypeptides, respectively.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1305 cm⁻¹ to 1200 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1305 cm⁻¹ to 1200 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and in the spectral range of 1305 cm⁻¹ to 1200 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹.

In certain embodiments, the optical value, particularly the sample optical value, is determined in the spectral range of 1570 cm⁻¹ to 1515 cm⁻¹, and in the spectral range of 1440 cm⁻¹ to 1340 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1570 cm⁻¹ to 1515 cm⁻¹, and in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1305 cm⁻¹ to 1200 cm⁻¹, and in the spectral range of 1440 cm⁻¹ to 1340 cm⁻¹.

In certain embodiments, the optical value, particularly the sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1440 cm⁻¹ to 1340 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1305 cm⁻¹ to 1200 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1305 cm⁻¹ to 1200 cm⁻¹, and in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1440 cm⁻¹ to 1340 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and in the spectral range of 1305 cm⁻¹ to 1200 cm⁻¹, and in the spectral range of 1440 cm⁻¹ to 1340 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1570 cm⁻¹ to 1515 cm⁻¹, and in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1570 cm⁻¹ to 1515 cm⁻¹, and an additional optical value, particularly an additional sample optical value, is determined in the spectral range of 1440 cm⁻¹ to 1340 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1305 cm⁻¹ to 1200 cm⁻¹, and in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1305 cm⁻¹ to 1200 cm⁻¹, and in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1440 cm⁻¹ to 1340 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1440 cm⁻¹ to 1340 cm⁻¹, and in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1440 cm⁻¹ to 1340 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1440 cm⁻¹ to 1340 cm⁻¹, and in the spectral range of 1570 cm⁻¹ to 1515 cm⁻¹, and in the spectral range of 1305 cm⁻¹ to 1200 cm⁻¹, and in the spectral range of 3500 cm⁻¹ to 3060 cm⁻¹.

In certain embodiments, the optical value, particularly the sample optical value, is determined in the spectral range of 1440 cm⁻¹ to 1340 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and in the spectral range of 1570 cm⁻¹ to 1515 cm⁻¹, and in the spectral range of 1305 cm⁻¹ to 1200 cm⁻¹, and in the spectral range of 1440 cm⁻¹ to 1340 cm⁻¹, and in the spectral range of 3500 cm⁻¹ to 3060 cm⁻¹.

In certain embodiments, the optical value, particularly the sample optical value, is determined in the spectral range of 1690 cm⁻¹ to 1620 cm⁻¹, particularly 1660 cm⁻¹ to 1628 cm⁻¹, and an additional optical value, particularly an additional sample optical value, is determined in the spectral range of 1570 cm⁻¹ to 1515 cm⁻¹, and an additional optical value, particularly an additional sample optical value, is determined in the spectral range of 1305 cm⁻¹ to 1200 cm⁻¹, and an additional optical value, particularly an additional sample optical value, is determined in the spectral range of 1440 cm⁻¹ to 1340 cm⁻¹,and an additional optical value, particularly an additional sample optical value, is determined in the spectral range of 3500 cm⁻¹ to 3060 cm⁻¹.

In certain embodiments, particularly with respect to the above mentioned embodiments, the sample spectrum is determined in the same manner as the optical value or sample optical value.

In certain embodiments, particularly with respect to the above mentioned embodiments, an additional spectrum is determined in the same manner as the additional optical value or additional sample value.

According to a preferred embodiment of the invention, the liquid sample is dried on a sample holder prior to the spectroscopy step.

Advantageously, liquid samples prepared from intact muscle meat, instead of wet, artificially processed minced meat are particularly suitable for spectral acquisition, particularly by FTIR spectroscopy.

Furthermore, drying the sample prior to the spectroscopy step reduces the water content of the sample allowing better analysis of the resulting spectra.

In certain embodiments, a liquid sample is obtained by applying pressure on the meat sample.

In certain embodiments, pressure is applied on the meat sample by squeezing or centrifuging the meat sample.

In certain embodiments, in the spectroscopy step a plurality of optical values is determined in the spectral range, wherein each of the optical values of the plurality is compared to a corresponding control optical value of the at least one control sample, and wherein particularly the plurality of optical values constitutes a spectrum within the certain spectral range.

In certain embodiments, a spectrum is determined from the liquid sample in the spectroscopy step and a derivative of the spectrum is calculated in the comparison step, particularly in the computing step.

In the context of the present specification, the term derivative is used in its meaning known in the art of mathematics. The term "derivative of a spectrum" denotes a derivative of a function, wherein the independent variable is a wavelength or a wave number, and wherein the dependent variable is an optical value, and wherein the optical values determined by a spectroscopy measurement of a sample are approximated by the function.

In particular, the n^{th} derivative of a spectrum is calculated, where n is a natural number.

In certain embodiments, the first, third, fourth or fifth derivative, preferably the second derivative is calculated.

In certain embodiments, a spectrum is determined from the liquid sample in the spectroscopy step and a vector normalization of the spectrum is performed in the comparison step, particularly in the computing step.

Therein, the vector normalization is performed as follows: A plurality of optical values is determined from a liquid sample, wherein each optical value is determined at a specific wave number. Subsequently, a function is provided, wherein the function comprises a plurality of x-values, wherein each x-value corresponds to a wave number, and a plurality of y-values, wherein each y-value corresponds to an optical value, and wherein the function describes the relationship between the x-values and the y-values. Furthermore, a mean value of the plurality of y-values is determined, and the mean value is subtracted from each y-value, resulting in a plurality of corrected y-values. Subsequently, the square of each corrected y-value is calculated, and the sum of all squares is determined. Finally, each corrected y-value is divided by the sum of all squares, resulting in a plurality of vector normalized y-values, wherein the vector normalized y-values and the wave numbers, at which the respective y-value were determined, constitute a vector normalized spectrum.

Advantageously, vector normalization allows better analysis of spectra, particularly comparison of spectra.

In certain embodiments, the method further comprises the steps of measuring at least one first spectrum of a control sample from fresh meat and at least one second spectrum of a control sample from frozen and thawed meat, comparing the at least one first spectrum and the at least one second spectrum, identifying at least one wave number, at which the first spectrum and the second spectrum differ, and measuring the sample optical value or an additional sample optical value of the liquid sample at the identified wave number or wave numbers.

In certain embodiments, the difference between two spectra is determined by statistical analysis, more particularly by calculating Pearson's product moment correlation coefficient, or by calculating a spectral distance (Naumann, Labischinski, and Giesbrecht, 'The Characterization of Microorganisms by FT-IR spectroscopy' in "Modern Techniques for Rapid Microbiological Analysis", W. H. Nelson (Ed.), VCH publishers, New York, pp.43-96, 1991) or by analysis of variance (ANOVA) pairwise comparison.

In the context of the present specification, the terms Pearson's product moment correlation coefficient and analysis of variance (ANOVA) pairwise comparison are used in their meaning known in the art of mathematics and statistics.

In certain embodiments, comparing the at least one first spectrum and the at least one second spectrum, and/or identifying at least one wave number, at which the first spectrum and the second spectrum differ, further comprises applying a statistical method, particularly covariance analysis, more particularly covariance analysis with a sequential forward selection search strategy.

In the context of the present specification, the term sequential forward selection search strategy describes a computational method, wherein a list of features is generated, wherein each feature represents a characteristic spectral range of wave numbers of a set of spectra. Features are then added to a list of selected features, according to a partial F-value of the selected features (Udelhoven, Novozhilov, & Schmitt, Chemometrics and Intelligent Laboratory Systems 66, 2003).

Therein, "a candidate feature is added to previously selected features, and the selection criterion, a partial F-value, is calculated to evaluate the discrimination power of the candidate feature. As soon as this has been done for all candidate features, the one with the highest score is joined to the set of selected features. The search procedure is stopped when the desired number of selected features is reached or the discriminating power of a new feature drops below a predefined statistical threshold".

In certain embodiments, at least one statistical method, particularly covariance analysis or principal component analysis (Wang et al., Appl. and Environmental Microbiology, 76(18), 6266-6276, 2010) and/or hierarchical cluster analysis, and/or spectral subtraction, and/or visual spectral analysis, is used to determine the characteristic spectral range of wave numbers.

In the context of the present specification, the term spectral subtraction designates subtracting from each optical value of a first spectrum an optical value of a second spectrum, wherein the respective subtracted optical values of the first spectrum and the second spectrum have been determined at the same wavelength or wave number and using the same parameters during data acquisition and processing.

In the context of the present specification, the term visual spectral analysis designates the visual observation of a plurality of spectra by an observer, particularly a person, and identifying features, which are different between spectra.

In certain embodiments, an artificial neural network is used to determine the similarity value in the comparison step, particularly in the computing step.

According to a preferred embodiment of the invention, the similarity value between sample optical values and control optical values is determined by an artificial neural network, which is trained using a plurality of control optical values of a first plurality of control samples prepared from fresh meat, and a plurality of control optical values of a second plurality of control samples prepared from frozen and thawed meat.

In certain embodiments, a multilayer perceptron using a Resilient Learning Algorithm is applied as an artificial neural network (Udelhoven, Novozhilov, & Schmitt, Chemometrics and Intelligent Laboratory Systems 66 (2003), 219-226) in the method disclosed herein.

In certain embodiments, the similarity value determined by the artificial neural network comprises a categorical variable having the values "fresh", or" frozen and thawed", or "no result", wherein the value "fresh" is determined if a sample is sufficiently similar to the first plurality of control samples prepared from fresh meat, and wherein the value "frozen and thawed" is determined if a sample is sufficiently similar to the second plurality of control samples prepared from frozen and thawed meat, and wherein the value "no result" is determined if no sufficient similarity between the sample and the control samples can be determined.

Due to the differences in sample preparation (using liquid samples), the inventors were able to assign polypeptide specific bands (wave numbers) related to fresh meat or frozen and thawed meat, while in the prior art only a statistical correlation could be found using a broad spectral range, whereby the skilled person was not able to assign any specific band to the mode of meat storage (fresh versus frozen/thawed). Thus, particularly due to the specific sample preparation, the inventors were able to show a new causative correlation between a specific spectral band to the mode of meat storage (fresh versus frozen/thawed).

### Description of the figures

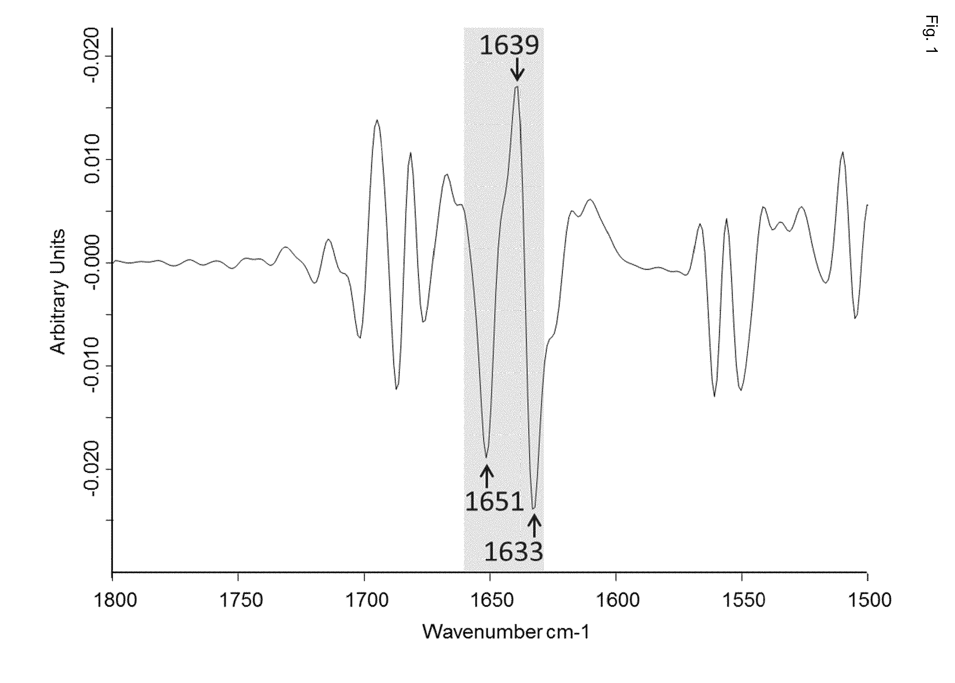

- Fig. 1: shows subtractive FTIR spectral analysis. Second derivate, vector-normalized FTIR average spectra were generated from fresh (refrigerated) and frozen/thawed chicken samples. Spectra from frozen/thawed chicken samples were subtracted from spectra of refrigerated chicken samples. The most profound differences were observed in the highlighted spectral range of 1,660 cm⁻¹ to 1,628 cm⁻¹, which can be assigned to the amide I band (1,670 cm⁻¹ to 1,625 cm⁻¹).

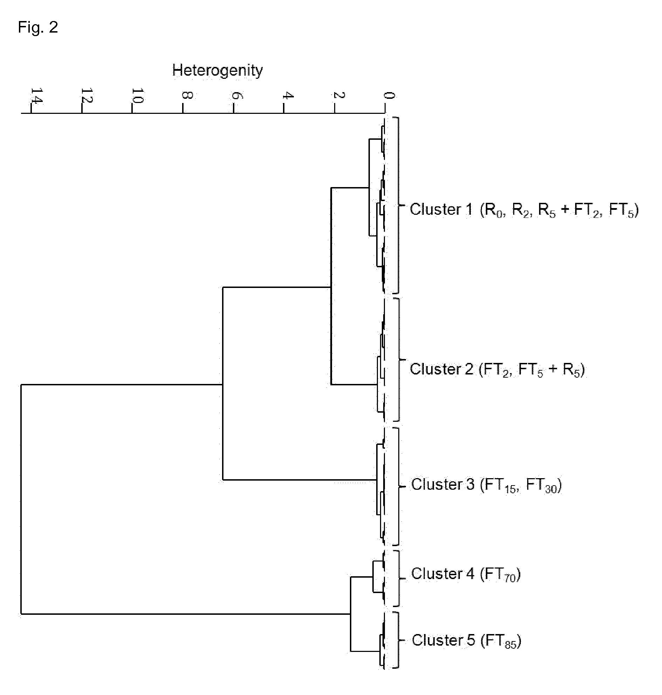

- Fig. 2: shows a dendrogram based on 108 FTIR spectra of fresh/refrigerated (R) and frozen/thawed (FT) chicken analyzed by hierarchical cluster analysis. While clusters 3 to 5 comprise FT samples only, clusters 1 and 2 are mixed clusters of spectra of both R and FT samples. Cluster 1 comprises predominantly spectra of R samples (R0, R2, R5), but also four spectra of FT samples (FT2, FT5). Cluster 2 comprises predominantly spectra of FT samples (FT2, FT5), but also five spectra of R samples (R5).

### Examples

The present invention provides proof of principle that FTIR and subsequent hierarchical cluster analysis (HCA) allow for differentiation of refrigerated chicken from chicken that had been subjected to frozen storage. It therefore represents a powerful tool that can be used by both retailers and governmental control agencies to ascertain correct labeling of chicken meat.

Particularly, the present invention provides the proof of principle that FTIR can be used to distinguish fresh and frozen/thawed chicken meat. Further, the use of improved pattern recognition algorithms such as trained artificial neuronal networks (Udelhoven, Novozhilov, & Schmitt, Chemometrics and Intelligent Laboratory Systems 66 (2003), 219-226) for analysis of FTIR spectra allows for identification of various previously frozen products, including beef, pork, lamb, and turkey.

### Materials and methods

### Chicken meat samples

An overview of all chicken meat samples used is provided in Table 1. A total of 16 samples of 50 g of chicken breast muscle were purchased from a local poultry meat producer (producer A). The fresh meat was immediately packed in plastic bags in line with the industry standard and vacuum-sealed. The cooled samples (4°C) were transported to the laboratory. The samples were randomly assigned to two groups: fresh samples that were only refrigerated (R; n = 6), and frozen/thawed samples (FT; n = 10). Refrigerated samples were kept at 4°C and were prepared for FTIR spectroscopy immediately (TO) and after 2 and 5 days. The frozen/thawed samples were stored at -20°C for 2, 5, 15, 30, 60, and 70 days and were gently thawed at 4°C for 6-7 h prior to sample preparation for FTIR spectroscopy. Two samples from a different poultry meat producer (producer B) were used for validation and were frozen for 85 days prior to sample preparation for FTIR spectroscopy.

**Table 1: Random assignment of chicken meat samples into two groups: fresh samples that were refrigerated only (R, n = 6); and frozen/thawed samples (FT, n = 14). Two samples per group and time point were analyzed in triplicates of 1:10 and 1:15 dilutions to generate a total of 108 FTIR spectra.**

| **Group** | **Sample ID** | **Storage duration** | **Storage conditions** | **Number of samples** | **Sample source** |
|---|---|---|---|---|---|
| R | R₀ | 0 days | 4°C | 2 | Producer A |
| | R₂ | 2 days | 4°C | 2 | Producer A |
| | R₅ | 5 days | 4°C | 2 | Producer A |
| | FT₂ | 2 days | -20°C | 2 | Producer A |
| | FT₅ | 5 days | -20°C | 2 | Producer A |
| | FT_{15d} | 15 days | -20°C | 2 | Producer A |
| | FT_{30d} | 30 days | -20°C | 2 | Producer A |
| | FT_{70d} | 70 days | -20°C | 2 | Producer A |
| | FT_{85d} | 85 days | -20°C | 2 | Producer B |

### Sample preparation and FTIR spectroscopy.

46.9 ± 1.09 (mean ± standard deviation) grams of the meat samples were transferred to 50 mL polypropylene centrifuge tubes. Upon centrifugation at 15000 x g for 15 min at 4°C, the liquid sample was removed and triplicates of 1:10 and 1:15 dilutions in 0.9% NaCl were prepared for two samples of each group and time point. A volume of 30 µl of the liquid sample dilutions was spotted on a zinc selenite (ZnSe) optical plate and dried at 40°C for 30 min. Infrared spectroscopy absorption spectra were recorded using a HTS-XT microplate adapter coupled to a Tensor 27 FTIR spectrometer (Bruker Optics GmbH, Ettlingen, Germany). Spectral acquisition was performed in transmission mode in the spectral range of 4000 cm⁻¹ to 500 cm⁻¹ using the following parameters: 6 cm⁻¹ spectral resolution, zero-filling factor 4, Blackmann-Harris 3-term apodization and 32 interferograms were averaged with background subtraction for each spectrum (Grunert et al., PLoS One 9(12), 2014).

### Spectral processing and chemometrics

Spectral evaluation and processing were performed using the software OPUS 7.2 (Bruker Optics GmbH). Second derivatives of the original spectra with a 9-point Savitzky-Golay filter were calculated to increase spectral resolution and to minimize problems with baseline shifts. Subsequent vector normalization was performed for the whole spectral range to adjust biomass variations among different sample preparations (Grunert et al., *PLoS One* 9(12), 2014). Subtractive spectral analysis was used to define spectral ranges of critical relevance to the discrimination of the different experimental groups. An average spectrum was calculated from the recorded second derivative and vector normalized IR spectra of the R and FT group separately and differential spectra analysis was performed. The average spectrum of R samples was subsequently subtracted from the average spectrum of FT samples. Chemometric analysis was performed on preprocessed data employing unsupervised hierarchical cluster analysis (HCA). Unsupervised methods are not based on prior knowledge and allow reduction of data complexity, while maintaining most of the original variance (Wenning & Scherer, Appl. Microbiol. Biotechnol. 97(16), 2013). The spectral windows of the 'amide region', dominated by the amide I and amide II bands of proteins and peptides (1660 cm⁻¹ to 1628 cm⁻¹), were selected for Hierarchical Cluster Analysis (HCA), offering the maximum discriminatory power.

### Artificial Neural Network (ANN)

To develop and to validate the potential of an artificial neuronal network (ANN) for the discrimination between fresh and frozen/thawed chicken meat, the software Neuro Developer (version 2.5b; Synthon GmbH, Heidelberg, Germany) was used (Udelhoven, Novozhilov, & Schmitt, Chemometrics and Intelligent Laboratory Systems 66, 2003). Preprocessed spectra used for HCA (n=108) were subdivided into two groups: (1) 86 spectra served as a reference data set (n = eight to ten spectra/ group) and (2) 21 spectra (n = two to three spectra/ group) were used for internal validation. One spectrum was excluded based on an outlier analysis using the software Unscrambler X (CAMO Software, Oslo, Norway). The reference data set used to calibrate the model was randomly divided into a training set (n = six to eight spectra/ group) and a pre-validation set (n = two spectra/ group). To reduce the dimensionality in the spectral data set, the most discriminative wave numbers were identified prior to the training process using the multivariate COVAR algorithm of the Neuro Developer software (based on a covariance analysis combined with the sequential forward selection search strategy).

### Results

The inventors evaluated the performance of FTIR as a tool for the differentiation of fresh (refrigerated only) and frozen/thawed chicken meat. Differential spectral analysis revealed significant differences between R and FT samples within the protein region (1800 cm⁻¹ to 1500 cm⁻¹) at 1660 cm⁻¹ to 1628 cm⁻¹ (Fig. 1). Spectral alterations in this frequency area are primarily determined by the confirmation sensitive amide I band (1670 cm⁻¹ to 1625 cm⁻¹) principally based on symmetric stretching vibrations of the carbonyl (C=O) functional group. The amide I band is indicative for changes in the protein secondary structure including alterations in the backbone conformation and the hydrogen bonding pattern. Subtractive FTIR spectral analysis revealed changes between R and FT samples associated to α-helical (1651 cm⁻¹) and β-plated sheet (1639 cm⁻¹, 1633 cm⁻¹) protein secondary structures. This highly discriminatory spectral range (1660 cm⁻¹ to 1628 cm⁻¹) was further applied for chemometric analysis by HCA. While FTIR spectra of samples that had only been frozen for up to five days could not be distinguished by HCA from those of fresh chicken samples, longer periods of frozen storage (15, 30, 70, 85 days) could be clearly identified. A dendrogram depicting the clustering of the FTIR spectra is provided as Fig. 2.

The establishment of the ANN was based on the same second derivative, vector normalized spectra used for HCA. The ANN was trained with selected spectral signatures defined by the COVAR algorithm as input data (input neurons) paired with the predefined output classes fresh (refrigerated only) and frozen/thawed chicken meat (output neurons). To achieve an optimal network performance the input and hidden neurons were automatically adjusted during the iterative training process until the global error was at its minimum (Naumann D., 2000. Infrared spectroscopy in microbiology, p 102-131. In Meyers RA (ed), Encyclopedia of analytical chemistry. John Wiley & Sons Ltd, Chichester, United Kingdom).

The ANN training resulted in a single-level ANN using the 22 most discriminative wave numbers (input neurons), two hidden neurons, and two output neurons. Two to three randomly selected spectra of each of the nine sample groups (R_{0,2,5} and FT₂,_{5,15,30,75,85}) were used for internal validation. A correct classification was achieved for 20 out of 21 samples. One sample (FT₈₅) yielded an ambiguous result.

Advantageously, FTIR spectroscopy-based differentiation of fresh and frozen/thawed meat requires only 1 hour analysis time in total (including sample preparation and FTIR spectroscopy) and less than 5 minutes 'hands-on time' for preparation per sample. In addition, only a minimum of 5 µL liquid sample per measurement is required. Operating expenses for running the FTIR spectroscopy measurement are low because no further reagents are required and sample holders (ZnSe plates) are reusable.

## Claims

1. A method for analysing a meat sample, wherein the method comprises:
a. providing a meat sample, and obtaining a liquid sample from said meat sample, and
b. measuring a sample optical value of said liquid sample in a spectral range of 4000 cm⁻¹ to 400 cm⁻¹ in a spectroscopy step, and
c. determining, in a comparison step, particularly in a computing step, a similarity value by comparing said sample optical value to at least one control optical value obtained from at least one control sample, wherein said at least one control sample has been prepared from fresh meat and/or from frozen and thawed meat, and
d. assigning said meat sample to "fresh meat" or "frozen and thawed meat" based on said similarity value.

2. The method according to claim 1, **characterized in that** said optical value is an absorption value of said liquid sample.

3. The method according to any one of the claims 1 to 2, **characterized in that** said optical value is obtained by Fourier-transformed infrared (FTIR) spectroscopy or Attenuated Total Reflection (ATR) spectroscopy.

4. The method according to claim 1, **characterized in that** said optical value is a scattering value of said liquid sample, wherein said spectral range from 4000 cm⁻¹ to 400 cm⁻¹ refers to the Raman shift of the inelastically scattered light.

5. The method according to any one of the preceding claims, **characterized in that** said spectral range is 3800 cm⁻¹ to 400 cm⁻¹, particularly 1800 cm⁻¹ to 1000 cm⁻¹, and/or 3500 cm⁻¹ to 3060 cm⁻¹.

6. The method according to any one of the preceding claims, wherein said spectral range is 1690 cm⁻¹ to 1620 cm⁻¹, particularly 1660 cm⁻¹ to 1628 cm⁻¹, and/or 1570 cm⁻¹ to 1515 cm⁻¹, and/or 1305 cm⁻¹ to 1200 cm⁻¹, and/or 1440 cm⁻¹ to 1340 cm⁻¹, and/or 3500 cm⁻¹ to 3060 cm⁻¹.

7. The method according to any one of the preceding claims, **characterized in that** said spectral range is 1690 cm⁻¹ to 1620 cm⁻¹, particularly 1660 cm⁻¹ to 1628 cm⁻¹, and/or 1570 cm⁻¹ to 1515 cm⁻¹, and/or 1440 cm⁻¹ to 1340 cm¹, and/or 1305 cm⁻¹ to 1200 cm⁻¹.

8. The method according to any one of the preceding claims **characterized in that** said liquid sample is dried on a sample holder prior to said spectroscopy step.

9. The method according to any of the preceding claims, **characterized in that** in said spectroscopy step a plurality of optical values is determined in said spectral range, wherein each of said optical values of said plurality is compared to a corresponding control optical value of said at least one control sample.

10. The method according to any one of the preceding claims, **characterized in that** a spectrum is determined from said liquid sample in said spectroscopy step, and a derivative of said spectrum is calculated in said comparison step, particularly in said computing step.

11. The method according to any one of the preceding claims, **characterized in that** a spectrum is determined from said liquid sample in said spectroscopy step, and a vector normalization of said spectrum is performed in said comparison step, particularly in said computing step.

12. The method according to any one of the preceding claims, further comprising the steps of:
• measuring at least one first spectrum of said control sample from fresh meat and at least one second spectrum of said control sample from frozen and thawed meat,
• comparing said at least one first spectrum and said at least one second spectrum,
• identifying at least one wave number, at which said first spectrum and said second spectrum differ, and
• measuring said sample optical value or an additional sample optical value of said liquid sample at said identified wave number or wave numbers.

13. The method according to claim 12, **characterized in that** comparing said at least one first spectrum and said at least one second spectrum, and/or identifying at least one wave number, at which said first spectrum and said second spectrum differ, further comprises applying a statistical method, particularly covariance analysis, more particularly covariance analysis with a sequential forward selection search strategy.

14. The method according to any one of the preceding claims, **characterized in that** an artificial neural network is used to determine said similarity value in said comparison step, particularly in said computing step.
